# EUROPEAN PATENT SPECIFICATION

(11) **EP 0 502 485 B1**
(45) Date of publication and mention of the grant of the patent: **23.10.1996**
(21) Application number: 92103639.8
(22) Date of filing: 03.03.1992
(51) Int. Cl.: A61M 3/02

(54) **Device for the lavage, for therapeutic or hygiene purposes, of human and animal internal ducts**
Vorrichtung für die Wäsche, aus therapeutischen oder hygienischen Gründen, von menschlichen oder tierischen internen Leitungen
Dispositif pour le lavage, pour des raisons thérapeutiques ou d'hygiène, des conduites internes humaines ou des animaux

(30) Priority: 06.03.1991 IT MI910582
(43) Date of publication of application: 09.09.1992
(73) Proprietor: D.D. S.r.l., I-20123 Milan (IT)
(72) Inventor: Apolet, Josef, I-20144 Milan (IT); Ballotta, Romano, I-43019 Soragna (Parma) (IT); Cavallo, Giovanni, I-15011 Acqui Terme (Alessandria) (IT)
(74) Representative: Gervasi, Gemma, Dr.

(56) References cited:
- EP-A- 0 313 684
- GB-A- 0 006 842
- US-A- 1 503 579
- US-A- 2 888 925

## Description

The present invention relates to a device suitable for the introduction of lavage liquids, for hygiene or therapeutic purposes, in internal ducts of the human or animal body, according to the preamble of claim 1, which is based upon GB-A-6842.

### PRIOR ART

It is known that, in case of particular affections of internal organs, such as the auricular, vaginal, uterine, anal, ducts, etc. medicaments have to be administered in the form of solutions for the irrigation of the duct, and that, also for a common preventive and hygiene praxis, the lavage of said internal parts should be performed regularly.

The instruments normally employed, continuous suppliers such as phleboclysis, syringes or other plunger instruments, rubber syringes, etc. have normally a container in which is contained, or is taken in at the time of use, the liquid to be employed, which is then administered through a suitable duct.

Obviously, with devices of that type it is necessary to repeatedly stop the lavage operations in order to allow the aspiration, or in general the outpouring of the introduced liquid from the duct which is being washed. This is evidently inconvenient for the operator and requires for the patient the presence of specialised personnel in order that the operation be safely performed.

In the art are also well known devices for irrigation of internal organs, such as a vagina or an abdominal cavity. For instance, US-A-1 503 579 (DEWEY) relates to vaginal syringes of the plug type, and its principal objects are first to provide for keeping cool the parts of said syringe which should be kept cool when a hot douche is used; second, to provide for a sanitary device which can be cleaned and sterilized. A canal is provided with inflow openings, whereas the drainage of the used liquid is performed by an outflow canal.

GB-A-6842 (SPECK) and US-A-2 888 925 (PHILIPS) show devices similar to the prior one. In particular, they relate to syringes adapted for cleaning the human vagina. In GB-A-6842 (SPECK) the evacuation canal is a central one, whereas the inflow openings for the introduction of the clean liquid are peripherally disposed around said evacuation canal. On the contrary, in US-A-2 888 925 (PHILIPS) the discharge nozzle for the cleansing liquid is surrounded by a hollow body provided with return feed holes in order to drain the used liquid off.

EP-A-0 313 684 (GENECO) describes a catheter apparatus particularly adapted for implementing of the irrigation of the abdominal cavity.

This apparatus comprises an irrigation canal having an aperture which provides a flow communication with the bladder. A drainage canal having a plurality of apertures is also provided.

With these devices it is extremely difficult to perform cleansing of the ear duct because of the short circuit of the liquid between the inflow openings and the outflow canal. This is because the outcoming liquid through the canal works in order to suck incoming liquid from the ear duct. Therefore, effective cleaning cannot be done.

Furthermore, these documents fail to disclose, teach or suggest an ear duct irrigation device as claimed in the present application.

In order to overcome the drawbacks set forth above, the present invention provides for a device for the lavage of a human internal duct allowing at the same time the drainage of the lavage liquid, consisting of a body of substantially conical shape, which comprises connecting means of the body to a reservoir for the distribution of the lavage liquid, an input canal through which the lavage liquid is introduced and an output canal for the drainage of the used liquid.

The device is characterized by the fact that at least a part of the input canal for injection of the lavage liquid into an ear duct is inclined relative to the central axis of the body to direct impingement of the lavage liquid against wall surfaces of the ear duct without direct impingement of the stream against the eardrum.

### DETAILED DESCRIPTION OF THE INVENTION

The present invention allows to obviate the mentioned inconveniences by means of a device to be applied to the liquid supplying means, and in which a passage is provided through which the injected liquid may be drained out, thus permitting the performance of the dressing and/or lavage operations in a continuous and safe way.

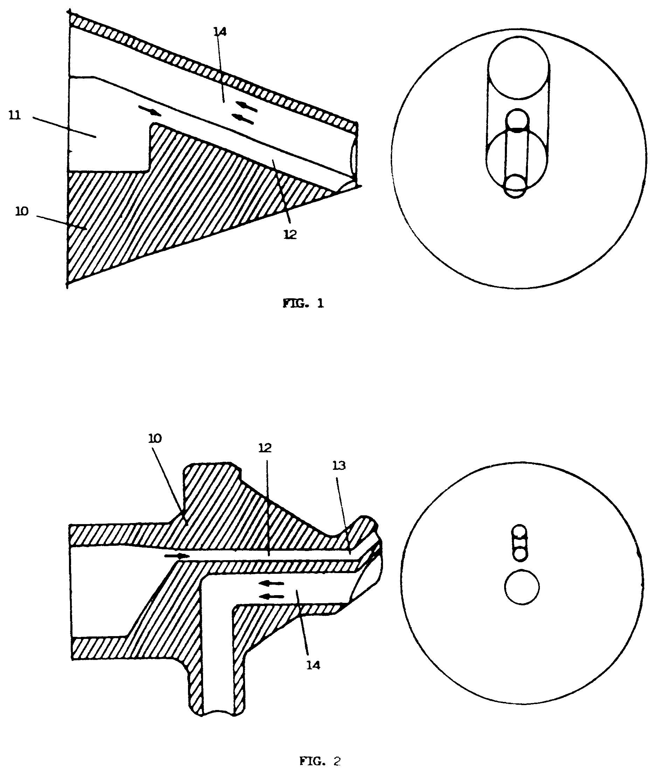

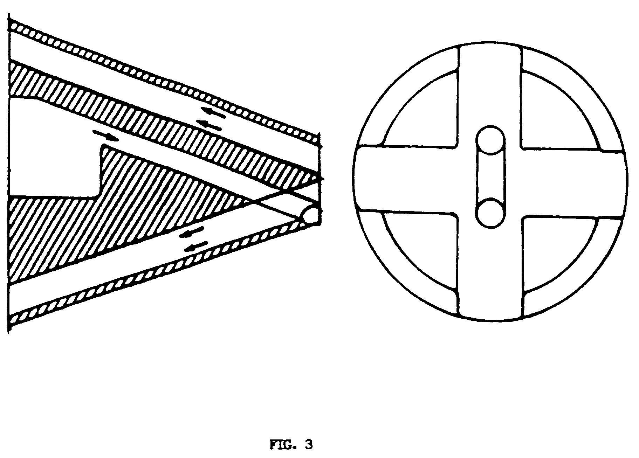

The invention will now be described with reference to the attached figures 1+3, which are shorn only for illustrative, non limitative purposes.

Figure 1 represents a section through the device according to the invention.

Figure 2 represents a particular embodiment of the invention.

Figure 3 represents, in section, some of the possible embodiments for the discharge channel.

As shown in figure 1, the device according to the invention consists of a body 10 of essentially conical shape apt to be applied to the human body orifice of the duct through which the lavage operation has to be performed.

According to a particular embodiment of the invention, in case the device should be employed for auricular lavage, the body 10 may be made to adapt to the shape of the patient's ear, following the techniques commonly employed for taking the auricular prints (as it is done for instance when adapting to the ear devices for improving the hearing). The injection and drainage ducts are then adapted to the device as will be described further below.

Said body 10 comprised a cavity 11 suitably shaped for connection (screwing, pressure, release connections, etc.) of the device to the liquid delivery means. From the cavity 11 a canal 12 goes through the body 10 reaching the external side opposite to the cavity 11.

Preferably the canal 12 is suitably shaped so that the injected liquid is deviated with respect to the central axis of the body 10. This may be obtained either by inclining the canal 12 with respect of the central axis of the body 10 (see fig.1) or bending it (see 13 in fig. 2).

In this way, the injected liquid is projected against the sides of the duct rather than directly against its end surface. This avoids exerting a pressure, which may be damaging, directly on internal organs which may be sensitive, as for instance the tympan membrane. The canal 12 may have any section, but preferably a cylindrical one; it may further be shaped or be provided with suitable deflectors of the liquid jet in order to impart to the liquid in the duct a helicoidal flow, which may mechanically facilitate the removal of matter to be washed out (sebum, mucilages, etc.).

A drainage canal 14 in the body 10 allows to drain out the lavage liquid which may be collected, if preferred, in a suitable container for analysis or other purposes.

Canal 14 may thus be an additional canal, completely independent of injection canal 12, or other similar arrangements may be foreseen.

According to a preferred embodiment, one may, for instance, foresee a single canal longitudinally divided by a separating wall into two sections through one of which flows the entering liquid and through the other the liquid discharged, or two coaxial canals, one for the input liquid and the other for the output liquid.

Fig.3 shows, in section, some possible forms. Others may be easily conceived by the expert.

The ratio between the section of the drainage canal and of the injection canal should be 1:1 or higher in order for the liquid to flow out freely.

According to a particular embodiment of the invention, the drainage canal is suitably shaped, (or comprises in its internal mouth a suitably shaped nozzle), and has multiple holes, to avoid clogging during the operation by the extracted material.

The different elements of the device, according to the invention, may be made out of any suitable substance.

Silicone and/or plastic materials are preferred, which allow a rapid and accurate cleaning and have an elasticity which permits to adapt the device to the body parts interested.

As the device, according to the invention, is of simple use, it may be included in a kit for personal use, consisting of one or more containers for the lavage liquid and of one or more devices, according to the invention, which could be in case thrown away after use.

The device according to the invention may also be connected to a suitable reservoir from which the liquid to be employed may be supplied by conventional means, e.g. plunger instruments or rubber syringes, etc.

## Claims

1. Device for the lavage of a human internal duct allowing at the same time the drainage of the lavage liquid, consisting of a body (10) of substantially conical shape, which comprises connecting means of said body to a reservoir for the distribution of the lavage liquid, an input canal (12) through which the lavage liquid is introduced and an output canal (14) for the drainage of the used liquid, device characterized in that at least a part of said input canal (12) for injection of the lavage liquid into an ear duct is inclined relative to the central axis of said body (10) to direct impingement of the lavage liquid against wall surfaces of said ear duct without direct impingement of the stream against the eardrum.

2. Device according to claim 1, characterized in that only one canal is present, divided by a separating wall into two sections, one (12) for the liquid input, the other (14) for the liquid output.

3. Device according to claim 1, characterized in that the ratio between the output canal (14) section and the input canal (12) section is at least 1 : 1 or higher.

4. Device according to claim 1, characterized in that the input canal (12) is shaped as to impress a helicoidal movement to the injected liquid.

5. Device according to claim 1, characterized in that said conical body (10) is suitably shaped as to adapt to the particular conformation of the patient auricle.

6. Device according to claim 1, characterized in that said conical body (10) consists of plastic and/or silicone materials.

7. Device according to claim 1, characterized in that said conical body (10) is firmly attached to said reservoir.

## Patentansprüche

1. Vorrichtung für die Wäsche von menschlichen internen Leitungen, indem sie gleichzeitig die Drainage der Wäscheflüssigkeit erlaubt, die vom einem kegelförmigen Körper (10) gebildet ist, der Verbindungsmittel vom obengenannten Körper mit einem Behälter für die Verteilung der Wäscheflüssigkeit, einen Eintrittskanal (12) für die Einführung der Wäscheflüssigkeit und einen Austrittskanal (14) für die Drainage der benutzten Flüssigkeit umfaßt, dadurch gekennzeichnet, daß mindestens einen Teil vom obegenannten Eintrittskanal (12) für die Einführung der Wäscheflüssigkeit in einen Gehörgang hinsichtlich der Zentralachse vom obengenannten Körper (10) geneigt ist, um die Wäscheflüssigkeit direkt gegen die Wände vom obengenannten Gehörgang zu richten ohne das Trommelfell direkt zu schlagen.

2. Vorrichtung nach Anspruch 1, dadurch gekennzeichnet, daß nur ein Kanal vorhanden ist, der von einer Trennwand in zwei Teilen getrennt ist, indem ein Teil (12) für den Eintritt der Flüssigkeit und der andere Teil (14) für den Austritt der Flüssigkeit ist.

3. Vorrichtung nach Anspruch 1, dadurch gekennzeichnet, daß das Verhältnis zwischen dem Schnitt des Austrittskanals (14) und dem Schnitt des Eintrittskanal (12) mindestens 1:1 oder höher ist.

4. Vorrichtung nach Anspruch 1, dadurch gekennzeichnet, daß der Eintrittskanal (12) eine bestimmte Form hat, um auf die eingespritzten Flüssigkeit eine spiralförmige Bewegung zu übertragen.

5. Vorrichtung nach Anspruch 1, dadurch gekennzeichnet, daß der obengenannte kegelförmige Körper (10) eine bestimmte Form hat, um sich der besonderen Form der Patientshörmuschel anzupassen.

6. Vorrichtung nach Anspruch 1, dadurch gekennzeichnet, daß der obengenannte kegelförmige Körper (10) aus Kunststoff und/oder Silikon besteht.

7. Vorrichtung nach Anspruch 1, dadurch gekennzeichnet, daß der obengenannte kegelförmige Körper (10) dem obengenannten Behälter fest verbunden ist.

## Revendications

1. Dispositif pour le lavage des conduites internes humaines qui permet au même temps le drainage du liquide de lavage formé par un corps (10) de forme conique, comprenant moyens de jonction du susdit corps à un réservoir pour la distribution du liquide de lavage, un canal de entrée (12) à travers le quel on introduit le liquide de lavage, et un canal de sortie (14) pour le drainage du liquide usé, dispositif caracterisé en ce que au moins une partie du susdit canal de entrée (12) pour l'introduction du liquide de lavage dans le conduit auditif est incliné par rapport à l'axe central du susdit corps (10) pour diriger le lancement du liquide de lavage contre les parois du susdit conduit auditif sans frapper directement le tympan.

2. Dispositif selon la revendication 1, caracterisé en ce que il y a seulement un canal, divisé par un paroi de séparation dans deux parties, l'une (12) pour l'entrée du liquide, et l'autre (14) pour la sortie du liquide.

3. Dispositif selon la revendication 1, caracterisé en ce que le rapport entre la section du canal de sortie (14) et celle du canal de entrée (12) est au moins 1:1 ou plus grande.

4. Dispositif selon la revendication 1, caracterisé en ce que le canal de entrée (12) a une forme apte à imprimer un mouvement hélicoïdal au liquide injecté.

5. Dispositif selon la revendication 1, caracterisé en ce que le susdit corps conique (10) a une forme apte à s'adapter à la particulière conformation du pavillon de l'oreille du patient.

6. Dispositif selon la revendication 1, caracterisé en ce que le susdit corps conique (10) est fait en plastique et/ou silicone.

7. Dispositif selon la revendication 1, caracterisé en ce que le susdit corps conique (10) est bien attaché au susdit réservoir.
